(19) Europäisches Patentamt
European Patent Office
Office européen des brevets

(11) Veröffentlichungsnummer: **0 265 933 B1**

(12) **EUROPÄISCHE PATENTSCHRIFT**

(45) Veröffentlichungstag der Patentschrift: **03.02.93**

(51) Int. Cl.⁵: **C12Q 1/60**, C12Q 1/26, C12Q 1/44, G01N 33/92

(21) Anmeldenummer: **87115841.6**

(22) Anmeldetag: **28.10.87**

Die Akte enthält technische Angaben, die nach dem Eingang der Anmeldung eingereicht wurden und die nicht in dieser Patentschrift enthalten sind.

(54) **Verfahren und Reagenz zur spezifischen Bestimmung des Cholesterins der HDL-Fraktion.**

(30) Priorität: **29.10.86 DE 3636851**

(43) Veröffentlichungstag der Anmeldung:
**04.05.88 Patentblatt 88/18**

(45) Bekanntmachung des Hinweises auf die Patenterteilung:
**03.02.93 Patentblatt 93/05**

(84) Benannte Vertragsstaaten:
**AT BE CH DE ES FR GB GR IT LI LU NL SE**

(56) Entgegenhaltungen:
**EP-A- 0 044 432**
**EP-A- 0 053 692**
**EP-A- 0 088 420**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Kerscher, Lorenz, Dr.**
**Paul Loebe-Str. 21**
**W-8122 Penzberg(DE)**
Erfinder: **Pautz, Brigitte**
**Kientalstrasse 27**
**W-8036 Herrsching(DE)**
Erfinder: **Trunk, Gisela**
**Schönbichlstrasse 49**
**W-8121 Wielenbach(DE)**
Erfinder: **Ziegenhorn, Joachim, Dr.**
**Ina-Seidel-Weg 1**
**W-8130 Starnberg(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

**EP 0 265 933 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren und ein Reagenz zur spezifischen Bestimmung des Cholesterins der HDL-Fraktion in Gegenwart der LDL-Fraktion der Lipoproteine des Serums durch Einwirkung von Cholesterinesterase zur Freisetzung des Cholesterins und Oxidation des freigesetzten Cholesterins mit Cholesterinoxidase und Sauerstoff unter Bildung von $H_2O_2$ und kinetische Messung der $H_2O_2$-Bildung oder des Sauerstoffverbrauchs.

Die Bestimmung des Gesamtcholesterins sowie der einzelnen Cholesterin enthaltenden Lipidfraktionen im Serum hat im klinisch-chemischen Labor eine große Bedeutung. Hypercholesterinämie und Hypertriglyceridämie begünstigen die Entstehung der Atherosklerose und des Herzinfarktes. Das individuelle Koronarrisiko kann daher über die Bestimmung von Cholesterin und Triglyceriden im Serum erfaßt werden. Noch bessere Aussagen können gemacht werden, wenn außerdem auch die pathologischen Verschiebungen im Lipoproteinmuster ermittelt werden. Der Anteil der einzelnen Lipidfraktionen am Gesamtcholesterin läßt Aussagen über das Infarktrisiko zu, wobei einerseits umfangreiche klinische Studien erwiesen haben, daß ein erhöhter Serumgesamtcholesterinspiegel zu einem gesteigerten Herzinfarktrisiko führt, während andererseits eine inverse Beziehung zwischen dem Serum-HDL-Cholesterin und der Gefahr atherosklerotischer Blutgefäßveränderungen besteht. Die Lipoproteine des Serums lassen sich in 4 Gruppen einteilen : Chylomikronen, Prä-$\beta$-Lipoprotein, auch Very low density lipoprotein oder VLDL genannt; $\beta$-Lipoprotein, auch Low density lipoprotein oder LDL genannt und $\alpha$-Lipoprotein, auch High density lipoprotein oder HDL genannt.

Für die Bestimmung des Serumgesamtcholesterins stehen seit etwa 15 Jahren einfache, aber dennoch sehr genaue vollenzymatische Analysenverfahren zur Verfügung, die sowohl manuell als auch gleichermaßen gut an automatisierten Analysensystemen ohne jegliche Probenvorbereitung durchführbar sind und üblicherweise nach dem Reaktionsschema:

1.

$$\text{Cholesterinester} + H_2O \xrightarrow{\text{Cholesterinesterase}} \text{Cholesterin} + \text{Fettsäure}$$

2.

$$\text{Cholesterin} + O_2 \xrightarrow{\text{Cholesterinoxidase}} \Delta\,4\text{-Cholesten-3-on} + H_2O_2$$

ablaufen. Das gebildete $H_2O_2$ kann dann, bevorzugt colorimetrisch, ggf. kinetisch gemessen werden.

Es ist auch bereits bekannt, die Bestimmung des HDL-Cholesterins im Serum nach Abtrennung der anderen cholesterinhaltigen Lipoproteinpartikel durchzuführen. Die Abtrennung dieser Lipoproteine kann durch Ultrazentrifugation, Elektrophorese oder diverse Präzipitationsmethoden, wie Immunpräzipitation, Fällung mit Phosphorwolframsäure/$Mg^{2+}$, Dextransulfat/$Mg^{2+}$ oder Heparin/$Mn^{2+}$ oder mit Polyethylenglykol erfolgen. Alle diese Verfahren sind aufwendig und erfordern mehrere Reaktionsschritte und einen relativ großen Zeitaufwand.

Aus DE-OS 32 08 253.3 ist es weiterhin bekannt, daß der Cholesterinanteil der LDL-Fraktion direkt zu bestimmen ist. Diese Bestimmung beruht auf der Erkenntnis, daß unter bestimmten Bedingungen die Reaktionsgeschwindigkeit des Cholesterinanteils in der LDL-Fraktion linear von deren Konzentration abhängt, und daß bei einer kinetischen Messung der dabei erhaltene Meßwert direkt proportional zur LDL-Cholesterinkonzentration im Reaktionsansatz ist. Dieses Verfahren ist sehr gut zur Bestimmung der LDL geeignet, eine Bestimmung des HDL-Anteils ist hiermit jedoch nicht möglich.

Es war nun Aufgabe der vorliegenden Erfindung, ein Verfahren und ein Reagenz zu schaffen, das die Bestimmung des HDL im Serum mit einfachen Mitteln und in einer Stufe erlaubt. Insbesondere sollte ein Verfahren zur Verfügung gestellt werden, mit Hilfe dessen gleichzeitig auch das Gesamtcholesterin bestimmt werden kann.

Diese Aufgabe wird gelöst durch ein Verfahren zur spezifischen Bestimmung des Cholesterins der HDL-Fraktion in Gegenwart der LDL-Fraktion der Lipoproteine des Serums durch Einwirkung von Cholesterinesterase zur Freisetzung des Cholesterins und Oxidation des freigesetzten Cholesterin mit Cholesterinoxidase und Sauerstoff unter Bildung von $H_2O_2$ und kinetische Messung der $H_2O_2$-Bildung oder des

Sauerstoffverbrauchs, das dadurch gekennzeichnet ist, daß man die Cholesterinesterase aus Pankreas verwendet und, daß die Messung innerhalb von 2 Minuten bis 15 Minuten nach dem Start der Oxidasereaktion bei einer Temperatur von 20 bis 40 °C während eines vorbestimmten Zeitintervalls durchgeführt wird und während der Messung in der Reaktionslösung eine Cholesterinesterase-Konzentration von 0,05 bis 30 U/ml, eine Cholesterinoxidase-Konzentration von 0,1 bis 50 U/ml, eine Konzentration eines Tensids der Gallensäurengruppe von 1,0 bis 20 mmol/1, eine Konzentration an nichtionischen Detergens von 0,1 bis 10 g/l und ein pH-Wert von 5 bis 9 eingehalten werden.

Überraschenderweise wurde festgestellt, daß die Umsetzung des Cholesterins der Serum-Lipoproteine mittels Cholesterinesterase und Cholesterinoxidase in Gegenwart geeigneter Detergenskombinationen in zwei Phasen verläuft. Die Reaktion verläuft zunächst mit einer Geschwindigkeit, die abhängig von der LDL-Cholesterin-Konzentration, aber unabhängig von der HDL-Cholesterin-Konzentration ist. Im weiteren Reaktionsverlauf wird dann von einem von den Reaktionsbedingungen abhängigen Zeitpunkt an die Reaktionsgeschwindigkeit von der HDL-Cholesterin-Konzentration abhängig. Die Umsetzung der HDL-Fraktionen setzt also mit zeitlicher Verzögerung ein. Bei geeigneter Wahl der Reaktionsbedingungen kann die HDL-Umsetzung zu einem Zeitpunkt beobachtet werden, zu dem LDL-Cholesterin schon weitgehend umgesetzt ist. Die Extinktionsänderung in einem entsprechend gewählten Zeitintervall wird dann der HDL-Cholesterin-Konzentration proportional. Auf diese Weise ist es möglich, mittels eines einfachen Verfahrens das HDL direkt in Gegenwart der anderen cholesterinhaltigen Lipidfraktionen zu bestimmen.

Wesentlich für das erfindungsgemäße Verfahren ist es, daß die Reaktionsbedingungen so eingestellt werden, daß die HDL-Umsetzung zu einem günstigen Zeitpunkt erfolgt.

Bei dem erfindungsgemäßen Verfahren wird zuerst durch Zugabe von Cholesterinesterase zu der Reaktionslösung die Freisetzung des Cholesterins bewirkt. Als geeignet hat sich Cholesterinesterase aus Pankreas (z. B. Rinder oder Schweinepankreas) erwiesen. Die Konzentration der Cholesterinesterase in der Reaktionslösung beträgt 0,05 bis 30 U/ml. Bevorzugt wird eine Cholesterinesterase-Konzentration von 0,05 bis 10 U/ml, besonders bevorzugt von 0,05 bis 1 U/ml, eingesetzt.

Der Maßeinheit U liegt für Cholesterin-Esterase folgende Definition zugrunde:
1 U ist die Enzymmenge, welche bei 37 °C 1 µmol Cholesteryllinoleat pro Minute umsetzt, wobei die Reaktionsmischung neben der Cholesterinesterase folgende Substanzen enthält:

| | |
|---|---|
| 0,2 mol/l | Kaliumphosphatpuffer pH 7,0 |
| 0,15 mol/l | Natriumchlorid |
| 4 g/l | Natriumcholat |
| 16 g/l | Thesit R |
| 2 g/l | Phenol |
| 0,3 g/l | 4-Aminoantipyrin |
| 20 U/ml | Peroxidase aus Meerrettich |
| 0,2 U/ml | Cholesterinoxidase aus Nocardia erythropolis |
| 0,45 g/l | Cholesteryllinoleat |

Das freigesetzte Cholesterin wird dann mit Cholesterinoxidase zu Cholestenon und $H_2O_2$ oxidiert. Die Cholesterinoxidase kann ebenfalls aus verschiedenen Organismen erhalten werden. Bevorzugt wird eine Cholesterinoxidase, die aus Brevibacterium,Nocardia oder Streptomyces erhalten wurde, eingesetzt. Die Konzentration der Cholesterinoxidase beträgt 0,1 bis 50 U/ml. Bevorzugt wird die Cholesterinoxidase mit einer Konzentration von 1 bis 30 U/ml, besonders bevorzugt von 5 bis 25 U/ml, eingesetzt.

Der Maßeinheit U liegt für Cholesterinoxidase folgende Definition zugrunde: 1 U ist die Enzymmenge, welche bei 25 °C 1 µmol Cholesterin pro Minute umsetzt, wobei die Reaktionsmischung neben dem Enzym folgende Substanzen enthält:

| | |
|---|---|
| 0,47 mol/l | Kaliumphosphatpuffer pH 7,5 |
| 8 g/l | Thesit R |
| 2,5 g/l | n-Propanol |
| 0,125 g/l | Cholesterin |

Wesentlich für das erfindungsgemäße Verfahren ist weiterhin, daß in der Reaktionslösung ein Tensid aus der Gallensäuregruppe vorhanden ist. Dieses Tensid ist in einer Konzentration von 1,0 bis 20 mmol/l vorhanden. Bevorzugt beträgt die Konzentration des Tensids der Gallensäuregruppe 1,5 bis 8 mmol/l.

Tenside der Gallensäuregruppe sind an sich bekannt. Bevorzugt werden die Derivate der Cholsäure verwendet, wobei das Natriumcholat besonders bevorzugt ist.

In der Reaktionslösung ist weiterhin ein nichtionisches Detergens vorhanden. Die Konzentration dieses nichtionisches Detergens beträgt 0,1 bis 10 g/l, bevorzugt 0,4 bis 4 g/l. Als nichtionisches Tensid wird bevorzugt ein Detergens, das Polyethylenoxidgruppen enthält, verwendet. Ebenfalls geeignet sind Detergenzien auf Zuckerbasis. Beispiele für nichtionische Detergenzien sind Alkyl- oder Alkaryl-polyethylenoxidether wie n-Dodecylpolyethylenglykolether, iso-Tridekanolpolyethylenoxidether und iso-Octylphenolpolyethylenoxidether.

In einer günstigen Ausführungsform wird das nichtionische Detergens nach der Zugabe von Oxidase, jedoch 1 bis 14 Minuten vor der Messung zugegeben.

Der pH-Wert der Reaktionslösung liegt im Bereich von 5 bis 9. Zur Einstellung des erforderlichen pH-Wertbereiches werden die in diesem Bereich wirksamen Puffersubstanzen zugesetzt. Die Art der verwendeten Puffersubstanz ist an sich nicht kritisch. Bevorzugt wird jedoch Tris/HCl-Puffer und insbesondere Phosphatpuffer verwendet.

Das erfindungsgemäße Verfahren wird bei einer Temperatur im Bereich von 20 bis 40 °C durchgeführt. Bevorzugt wird bei einer Temperatur zwischen 25 und 37 °C gearbeitet.

Bei Einhaltung aller erfindungswesentlichen Parameter kann die Messung des Cholesterins der HDL-Fraktion in dem Zeitbereich von 2 Minuten bis 15 Minuten nach dem Start der Oxidasereaktion erfolgen. Bevorzugt wird die Messung in dem Zeitbereich von 3 bis 10 Minuten nach dem Start der Oxidasereaktion durchgeführt.

Wenn die Messung der $H_2O_2$-Bildung unter weniger günstigen Reaktions- und Meßbedingungen erfolgen muß, kann die Umsetzung von Resten der LDL-Fraktion noch innerhalb des für die Messung ausgewählten Zeitintervalls erfolgen und könnte somit die Messung der HDL-Fraktion stören. Um diese Störung zu vermeiden, wird in einer weiteren Ausführungform des erfindungsgemäßen Verfahrens durch Zusatz von Anti-LDL-Antikörpern ein derartiger Spezifitätsverlust verhindert. Dieses Antiserum bringt die Umsetzung der LDL in einem frühen Stadium zum Erliegen. Hierzu können an sich bekannte Anti-LDL-Antikörper oder auch Anti-Apolipoprotein-B-Antikörper allein oder in Mischung verwendet werden. Diese Antikörper liegen bevorzugt in einer Konzentration von $10^{-6}$ bis $10^{-4}$ mmol/l als entfettete $\gamma$-Globulin oder IgG-Präparation vor.

Wie bereits oben ausgeführt, erfolgt bei dem erfindungsgemäßen Verfahren zuerst die Freisetzung des in der LDL-Fraktion enthaltenen Cholesterins, so daß das Meßsignal zuerst proportional dem Cholesterin-Gehalt der LDL-Fraktion ist. Es ist daher möglich, mit Hilfe des erfindungsgemäßen Verfahrens aus einem Testansatz sowohl LDL-Cholesterin als auch HDL-Cholesterin zu bestimmen. Da die Reaktionsgeschwindigkeit zunächst LDL-abhängig und später HDL-abhängig ist, kann innerhalb jeder Reaktionsphase das jeweils günstigste Zeitintervall für die Messung LDL- bzw. HDL-abhängiger Extinktionsänderungen gewählt werden. Dazu wird innerhalb der ersten Minute nach dem Start der Oxidasereaktion die $H_2O_2$-Bildung kinetisch bestimmt, als Maß für das in der LDL-Fraktion des Serums gebundene Cholesterin. Anschließend wird in dem Zeitintervall von 2 bis 15 Minuten nach dem Start der Oxidasereaktion die $H_2O_2$-Bildung kinetisch bestimmt als Maß für das in der HDL-Fraktion des Serums gebundene Cholesterin.

Weiterhin ist es gemäß einer günstigen Ausführungsform des erfindungsgemäßen Verfahrens möglich, nach der Messung des HDL-Cholesterins die Konzentration an Cholesterinesterase durch Zugabe einer zweiten Reagenzlösung so zu erhöhen, daß die Umsetzung des gesamten Cholesteringehaltes innerhalb kurzer Zeit vollständig abläuft. Es kann dann die Gesamtmenge an Cholesterin in der Probe bestimmt werden. Hierzu genügt es, zusätzlich, d.h. über die für die HDL-Cholesterinbestimmung vorgeschriebene Menge hinaus, Cholesterinesterase und gegebenenfalls -oxidase und/oder Tensid in hohen Konzentrationen zuzusetzen, sobald die HDL-Bestimmung beendet ist.

Ebenso kann einfach der Zusatz einer ausreichenden Menge eines, für die schnelle und voll Umsetzung des Gesamtcholesterins, geeigneten Reagenzes gemäß dem Stand der Technik erfolgen.

Mit dem erfindungsgemäßen Verfahren ist es somit möglich, in einer Probe parallel den Anteil an LDL-Fraktion, HDL-Fraktion sowie das Gesamtcholesterin zu bestimmen.

Die Bestimmung des freien Cholesterins mittels Cholesterinoxidase unter Bildung von $H_2O_2$ und Cholestenon und die kinetische Bestimmung der Veränderung eines Reaktionspartners dieser Oxidationsreaktion ist bekannt, beispielsweise aus der deutschen Auslegeschrift 25 58 536. Bevorzugt wird dabei gebildetes $H_2O_2$ unter Anwendung der, dem Fachmann geläufigen colorimetrischen Methoden der $H_2O_2$-Bestimmung nachgewiesen. Eine typische $H_2O_2$-Bestimmungsmethode, die für das Verfahren der vorliegenden Erfindung sehr geeignet ist, beruht auf der Farbreaktion von Phenol oder einem Phenolderivat mit 4-Aminoantipyrin oder einem Derivat davon, in Anwesenheit von Peroxidase. Diese Bestimmungen sind dem Fachmann geläufig und brauchen hier nicht näher beschrieben zu werden.

Ein weiterer Gegenstand der Erfindung ist ein Reagenz zur spezifischen Bestimmung des Cholesterins der HDL-Fraktion in Gegenwart der LDL-Fraktion der Lipoproteine des Serums enthaltend Cholesterinesterase, Cholesterinoxidase, Tensid der Gallensäuregruppen, nichtionisches Detergens, Puffer-pH 5 bis 9 und ein System zur photometrischen Bestimmung von $H_2O_2$, das dadurch gekennzeichnet ist, daß es die Cholesterinesterase in einer Konzentration von 0,05 bis 30 U/ml, die Cholesterinoxidase in einer Konzentration von 0,1 bis 50 U/ml, ein Tensid der Gallensäuregruppen in einer Konzentration von 1,0 bis 20 mmol/l und ein nichtionisches Detergens in einer Konzentration von 0,1 bis 10 g/l, jeweils bezogen aus die im Test eingesetzte Verdünnung, enthält.

Zur Durchführung des erfindungsgemäßen Verfahrens aus einem Analysenautomaten können die einzelnen Reaktionskomponenten auch auf oder in einem Trägermaterial imprägniert sein. Als Trägermaterial kann ein saugfähiges, quellfähiges oder filmbildendes Trägermaterial, z. B. ein für Teststreifen bekanntes Trägermaterial wie Papier und ähnliche Vliesmaterialien, beispielsweise Teebeutelpapier, verwendet werden. Dabei können die Reaktionskomponenten auf mehrere, in Kontakt stehende Träger verteilt sein.

In einer bevorzugten Ausführungsform enthält das erfindungsgemäße Reagenz zusätzlich noch Antikörper gegen LDL und/oder Apolipoprotein B.

Die Erfindung ermöglicht es, das in HDL enthaltende Cholesterin direkt, ohne vorherige Abtrennung der anderen Lipoproteinfraktionen, zu bestimmen. Weiterhin ist es möglich, gleichzeitig auch noch das in der LDL-Fraktion enthaltende Cholesterin und das im Serum enthaltende Gesamtcholesterin zu bestimmen. Das Verfahren ist einfach durchzuführen und ist automatisierbar.

Die Erfindung wird durch die folgenden Beispiele und Abbildungen weiter erläutert.

Die Abbildungen zeigen:

- Fig. 1: Reaktionsverlauf mit Proben unterschiedlicher HDL-Cholesterinkonzentration bei einer konstanten LDL-Cholesterinkonzentration von 84 mg/dl. HDL-Cholesterin in Kurve 1: 65,6 mg/dl, Kurve 2: 56,5 mg/dl, Kurve 3: 47,4 mg/dl, Kurve 4: 38,3 mg/dl, Kurve 5: 29,2 mg/dl.
- Fig. 2: Auswertung von Fig. 1 im Zeitintervall $\Delta t = 0 - 1$ min.
- Fig. 3: Auswertung von Fig. 1 im Zeitintervall $\Delta t = 5 - 6$ min.
- Fig. 4: Vergleich der HDL-Cholesterinbestimmung gemäß der Erfindung (ohne Zusatz von anti-Apo-$\beta$-Antikörpern) mit der Phosphowolfram-Fällungsmethode (PWS).
- Fig. 5: Vergleich der HDL-Cholesterinbestimmung gemäß der Erfindung (mit Zusatz von $8 \cdot 10^{-6}$ mol/l anti-Apo-$\beta$-Antikörper) mit PWS.

Beispiele:

**Beispiel 1**

Zwei Humanseren gleichen Gehaltes an LDL-Cholesterin, aber unterschiedlichen Gehaltes an HDL-Cholesterin werden in verschiedenen Verhältnissen gemischt, so daß sich eine Serie von Proben ergibt, welche steigende Mengen HDL-Cholesterin bei konstantem LDL-Cholesterin enthalten. Je 0,02 ml dieser Proben werden mit je 2,0 ml eines Reagenzes der folgenden Zusammensetzung bei 30 °C inkubiert:

| | |
|---|---|
| 0,1 M | Kaliumphosphatpuffer pH 6,7 |
| 8,6 mM | Tribromhydroxybenzoesäure |
| 1,6 mM | 4-Aminoantipyrin |
| 3 mM | Natriumcholat |
| 0,1 % | Polyethylenglycol 6000 |
| 0,1 % | Thesit$^R$ |
| 1 U/ml | Cholesterin-Esterase aus Schweinepankreas |
| 1 U/ml | Cholesterin-Oxidase aus Nocardia |
| 2,5 U/ml | Peroxidase |

Fig. 1 zeigt, daß die Extinktionszunahme bei 546 nm zunächst weitgehend von der HDL-Cholesterinkonzentration unabhängig ist, im weiteren Verlauf jedoch deutlich von ihr abhängig wird. Entsprechend ist die Extinktionszunahme während der ersten Minute kein Maß für die HDL-Cholesterinkonzentration (Fig. 2), während die Extinktionszunahme in der sechsten Minute der HDL-Cholesterinkonzentration proportional ist (Fig. 3).

**Beispiel 2**

Zwei Humanseren gleichen Gehalts an LDL-Cholesterin, aber unterschiedlichen Gehalts an HDL-Cholesterin werden in verschiedenen Verhältnissen gemischt, so daß sich eine Serie von Proben ergibt, welche steigende Mengen HDL-Cholesterin bei konstantem LDL-Cholesterin enthalten. Je 0,02 ml dieser Proben werden mit je 2,0 ml eines Reagenzes der folgenden Zusammensetzung bei 30 °C inkubiert:

| | |
|---|---|
| 0,1 M | Kaliumphosphatpuffer pH 6,7 |
| 8,6 mM | Tribromhydroxybenzoesäure |
| 1,6 mM | 4-Aminoantipyrin |
| 3 mM | Natriumcholat |
| 0,1 % | Polyethylenglycol 6000 |
| 0,1 % | Thesit$^R$ |
| 1 U/ml | Cholesterin-Esterase aus Schweinepankreas |
| 1 U/ml | Cholesterin-Oxidase aus Brevibacterium |
| 2,5 U/ml | Peroxidase |

Wie in Beispiel 1 ist auch hier die Extinktionszunahme bei 546 nm zunächst weitgehend von der HDL-Cholesterinkonzentration unabhängig und wird in der 6. Minute proportional der HDL-Cholesterinkonzentration.

**Beispiel 3**

Ausgehend von der in Beispiel 1 erläuterten Reaktionsmischung werden die Konzentrationen der folgenden Komponenten variiert:
Thesit$^R$, Natriumcholat, Cholesterin-Esterase, Cholesterin-Oxidase. Mit diesen Varianten werden die in Beispiel 1 geschilderten Untersuchungen durchgeführt. Die beobachteten Abhängigkeiten von der HDL-Konzentration sind in der vorliegenden Tabelle in Form der bei Auswertung verschiedener Zeitintervalle erhaltenen Regressionsdaten dargestellt.

| Variierter Einsatzstoff: | Günstiges Meßintervall | Korrelationskoeffizient | Regressionsgerade (x: theor. Wert, mg/dl) |
|---|---|---|---|
| Thesit$^R$: | | | |
| 0,4 g/l | 8-10 min | 0,998 | y = 0,91 + 2,9 |
| 1 g/l | 4-5 min | 0,996 | y = 1,22x - 5,6 |
| 4 g/l | 2-3 min | 0,999 | y = 0,68x + 9,4 |
| Natriumcholat: | | | |
| 1 mmol/l | 6-10 min | 0,988 | y = 1,08x + 0,2 |
| 3 mmol/l | 4-5 min | 0,996 | y = 1,22x - 5,6 |
| 5 mmol/l | 4-5 min | 0,997 | y = 0,89x + 4,9 |
| Cholesterin-Esterase: | | | |
| 0,3 U/ml | 6-7 min | 0,999 | y = 0,94x + 1,9 |
| 1 U/ml | 4-5 min | 0,996 | y = 1,22x - 5,6 |
| 3 U/ml | 4-5 min | 0,998 | y = 0,98x - 2,2 |
| Cholesterin-Oxidase: | | | |
| 0,3 U/ml | 5-6 min | 0,979 | y = 0,93x + 3,4 |
| 1 U/ml | 4-5 min | 0,996 | y = 1,22x - 5,6 |
| 3 U/ml | 5-6 min | 0,996 | y = 1,03x + 1,7 |

**Beispiel 4** (Vergleichsbeispiel)

Eine Reaktionsmischung enthält 0,5 U/ml Cholesterin-Esterase aus Pseudomonas und 2 U/ml Cholesterinoxidase aus Streptomyces; ansonsten entspricht die Zusammensetzung Beispiel 1. Auch hier beobachtet man in entsprechend gewählten Zeitintervallen eine ausgeprägte Abhängigkeit von der HDL-Cholesterin-Konzentration; in der 4. Inkubationsminute bei 37 °C erhält man z. B. bei einer Auswertung entsprechend Fig. 2 bzw. 3 eine Regressionsgerade y = 1,02x + 1,5 mg/dl. Ein ähnliches Resultat ergibt auch die Kombination von 1,5 U/ml Cholesterin-Esterase aus Candida cylindracea und 2 U/ml Cholesterin-Oxidase aus Streptomyces (y = 1,13x - 0,9).

**Beispiel 5**

Es wird vorgegangen, wie in Beispiel 1 erläutert, jedoch werden anstelle von Thesit$^R$ andere nichtionische Detergentien eingesetzt. Die beobachteten Abhängigkeiten von der HDL-Cholesterin-Konzentration sind in der folgenden Tabelle in Form der bei Auswertung verschiedener Zeitintervalle erhaltenen Regressionsdaten dargestellt.

| Detergens | Meßinter-vall | Korrelations-koeffizient | Regressionsgerade (x: theor.Wert, mg/dl) |
|---|---|---|---|
| Triton[R] X 100, 1 g/l | 5-6 min | 0,991 | y = 0,78x + 11,2 |
| " , 2 g/l | 4-5 min | 0,993 | y = 0,81x + 8,9 |
| Genapol[R] X 80, 0,6 g/l | 6-8 min | 0,987 | y = 0,95x - 2,2 |
| " , 1,5 g/l | 4-5 min | 0,995 | y = 0,86x - 3,6 |

**Beispiel 6**

An einem photometrischen Analysenautomaten kann man über die Extinktionsdifferenzen in günstig gewählten Zeitintervallen den HDL-Cholesteringehalt in Humanseren ermitteln. Die Kalibration erfolgt mittels eines Humanserums bekannten HDL-Cholesteringehalts. Das Reagenz entspricht grundsätzlich dem Reagenz in Beispiel 1, wobei der Fachmann den Test durch Variation der Aktivität von Cholesterin-Esterase und/oder Cholesterin-Oxidase, der Konzentration von Natriumcholat und Thesit[R], des Zeitintervalls sowie durch Zusatz von käuflichem Anti-B-Antiserum auf den zum Einsatz kommenden Analysenautomaten abstimmen kann. Fig. 4 zeigt, daß beispielsweise ähnliche Werte wie mit der etablierten Phosphowolframat-Fällungsmethode für HDL-Cholesterin erzielt werden können, wenn man die Aktivität der Cholesterin-Oxidase gegenüber Beispiel 1 auf 0,36 U/ml reduziert und bei 30 °C die Extinktionszunahme innerhalb der siebten Minute der Inkubation mißt. Fig. 5 zeigt, daß unter diesen Bedingungen ein Zusatz von 8 x $10^{-6}$ mol/l eines entfetteten Antikörpers gegen LDL die Übereinstimmung der Methoden noch weiter verbessert.
Regressionsgeraden (Standardhauptkomponentenanalyse):

Fig. 4: Y = 0,771 x + 3,551 (mg/dl)
Fig. 5: Y = 0,981 x + 0,034 (mg/dl)

**Beispiel 7**

Es wurde vorgegangen, wie in Beispiel 6 beschrieben. In parallelen Versuchen wurden unabhängig voneinander das Zeitintervall für die photometrische Messung und der Zusatz von entfettetem Anti-LDL-Antiserum variiert. Der Vergleich der für ein Kollektiv von Humanseren erhaltenen Daten mit den Resultaten der Phosphowolframsäure-Fällungsmethode ist in der folgenden Tabelle in Form der Regressionsdaten dargestellt.

| Konzentration Anti-Apo B-IgG | Meßintervall | Korrelationskoeffizient | Regressions- (x: PWS-Fällung, mg/dl) |
|---|---|---|---|
| $4 \cdot 10^{-6}$ | 3-4 min | 0,709 | y = 0,44x + 11,4 |
| | 4-5 min | 0,829 | y = 0,57x + 7,9 |
| | 5-6 min | 0,906 | y = 0,67x + 3,8 |
| | 6-7 min | 0,973 | y = 0,77x + 3,6 |
| | 9-10 min | 0,826 | y = 0,54x + 11,3 |
| $8 \cdot 10^{-6}$ | 3-4 min | 0,684 | y = 0,58x + 9,8 |
| | 4-5 min | 0,786 | y = 0,69x + 4,6 |
| | 5-6 min | 0,873 | y = 0,84x + 0,4 |
| | 6-7 min | 0,968 | y = 0,98x + 0,03 |
| | 9-10 min | 0,785 | y = 0,66x + 6,3 |
| $1{,}6 \cdot 10^{-5}$ | 3-4 min | 0,679 | y = 0,55x + 10,8 |
| | 4-5 min | 0,770 | y = 0,62x + 8,3 |
| | 5-6 min | 0,866 | y = 0,71x + 5,0 |
| | 6-7 min | 0,901 | y = 1,04x - 8,8 |
| | 9-10 min | 0,757 | y = 0,64x + 8,8 |

**Beispiel 8**

Durch geeignete Wahl des Meßintervalls kann bei allen in der klinischen Chemie gebräuchlichen Meßtemperaturen eine gute Übereinstimmung mit den Ergebnissen der etablierten Phosphowolframat-Fällungsmethode für HDL-Cholesterin erzielt werden. Für die im folgenden in Form der Regressionsdaten dargestellte Serie von Methodenvergleichen anhand eines Kollektivs von 27 Humanseren wurde folgende Ausführungsform des erfindungsgemäßen Verfahrens gewählt:

| | |
|---|---|
| Gerät: | Hitachi 705 |
| Meßwellenlänge: | 505 nm |
| Vergleichswellenlänge: | 600 nm |
| Kalibrator: | Humanserum bekannten HDL-Cholesterin-Gehalts |
| Probenvolumen: | 10 µl |
| Reagensvolumen: | 0,5 ml |

| Reagenszusammensetzung: | |
|---|---|
| 0,1 M | Kaliumphosphatpuffer pH 6,7 |
| 8,6 mM | Tribromhydroxybenzoesäure |
| 1,6 mM | 4-Aminoantipyrin |
| 5 mM | Natriumcholat |
| 0,1 % | Polyethylenglycol MG 6000 |
| 1,5 g/l | Thesit$^{R}$ (nichtionisches Detergens) |
| 0,25 U/ml | Cholesterin-Esterase aus Schweinepankreas Cholesterin-Oxidase wie unten angegeben |
| 2,5 U/ml | Peroxidase |

Seria a: 25 U/ml Cholesterinoxidase aus Nocardia

| Temperatur | Meßintervall | Korrelations-Koeffizient | Regressionsgerade (n=27, X: PWS-Fällung, mg/dl) |
|---|---|---|---|
| 25° C | 8-9 min<br>9-10 min | 0,954<br>0,966 | y=0,93 + 4,0<br>y=0,99 + 0,9 |
| 30° C | 4-5 min<br>5-6 min | 0,966<br>0,969 | y=0,97x + 0,9<br>y=1,06x - 2,1 |
| 37° C | 3-4 min<br>4-5 min | 0,961<br>0,896 | y=1,02x - 1,3<br>y=1,03x - 1,1 |

Serie b: 10 U/ml Cholesterinoxidase aus Brevibacterium

| Temperatur | Meßintervall | Korrelations-Koeffizient | Regressionsgerade (n=27, x: PWS-Fällung, mg/dl) |
|---|---|---|---|
| 25° C | 8-9 min<br>9-10 min | 0,807<br>0,858 | y=0,45x + 14,6<br>y=0,52x + 13,2 |
| 30° C | 7-8 min<br>8-9 min | 0,937<br>0,938 | y=0,93x + 4,1<br>y=0,88x + 4,0 |
| 37° C | 3-4 min<br>4-5 min | 0,929<br>0,914 | y=0,87x + 6,4<br>y=0,74x + 9,5 |

**Patentansprüche**

1. Verfahren zur spezifischen Bestimmung des Cholesterins der HDL-Fraktion in Gegenwart der LDL-Fraktion der Lipoproteine des Serums durch Einwirkung von Cholesterinesterase zur Freisetzung des Cholesterins und Oxidation des freigesetzten Cholesterins mit Cholesterinoxidase und Sauerstoff unter Bildung von $H_2O_2$ und kinetische Messung der $H_2O_2$-Bildung oder des Sauerstoffverbrauchs,
**dadurch gekennzeichnet,**
daß man die Cholesterinesterase aus Pankreas verwendet und daß die Messung innerhalb von 2 Minuten bis 15 Minuten nach dem Start der Oxidasereaktion bei einer Temperatur von 20 bis 40 °C während eines vorbestimmten Zeitintervalls durchgeführt wird und während der Messung in der Reaktionslösung eine Cholesterinesterasekonzentration von 0,05 bis 30 U/ml, eine Cholesterinoxidasekonzentration von 0,1 bis 50 U/ml, eine Konzentration eines Tensids der Gallensäuregruppe von 1,0 bis 20 mmol/l, eine Konzentration an nichtionischem Detergens von 0,1 bis 10 g/l und ein pH-Wert von 5 bis 9 eingehalten werden.

2. Verfahren nach Anspruch 1, **dadurch gekennzeichnet**, daß das nichtionische Detergens nach der Zugabe der Oxidase, jedoch 1 bis 14 Minuten vor der Messung zugegeben wird.

3. Verfahren nach Anspruch 1 oder 2, **dadurch gekennzeichnet,** daß die Messung innerhalb von 3 bis 10 Minuten nach dem Start der Oxidasereaktion durchgeführt wird.

4. Verfahren nach Anspruch 1 oder 2, **dadurch gekennzeichnet,** daß eine Cholesterinesterasekonzentration von 0,05 bis 10 U/ml eingehalten wird.

**5.** Verfahren nach Anspruch 1 oder 2, **dadurch gekennzeichnet,** daß eine Cholesterinoxidasekonzentration von 1 bis 30 U/ml eingehalten wird.

**6.** Verfahren nach Anspruch 1 oder 2, **dadurch gekennzeichnet,** daß eine Konzentration an Tensid der Gallensäuregruppe von 1,5 bis 8 mmol/l eingehalten wird.

**7.** Verfahren nach Anspruch 1 oder 2, **dadurch gekennzeichnet,** daß eine Konzentration an nichtionischem Tensid von 0,4 bis 4 g/l eingehalten wird.

**8.** Verfahren nach einem der vorhergehenden Ansprüche, **dadurch gekennzeichnet,** daß bei einer Temperatur von 25 bis 37° C gemessen wird.

**9.** Verfahren nach einem der vorhergehenden Ansprüche, **dadurch gekennzeichnet,** daß als Tensid der Gallensäuregruppe Natriumcholat verwendet wird.

**10.** Verfahren nach einem der vorhergehenden Ansprüche, **dadurch gekennzeichnet,** daß als nichtionisches Tensid ein Polyethylenoxidgruppen enthaltendes Tensid verwendet wird.

**11.** Verfahren nach einem der vorhergehenden Ansprüche, **dadurch gekennzeichnet,** daß die Umsetzung in Gegenwart von Anti-LDL-Antikörpern oder/und Anti-Apolipoprotein B-Antikörpern durchgeführt wird.

**12.** Verfahren nach einem der vorhergehenden Ansprüche, **dadurch gekennzeichnet,** daß zusätzlich innerhalb der ersten beiden Minuten nach dem Start der Oxidasereaktion die $H_2O_2$-Bildung kinetisch bestimmt wird als Maß für das in der LDL-Fraktion des Serums gebundene Cholesterin.

**13.** Verfahren nach einem der vorhergehenden Ansprüche, **dadurch gekennzeichnet,** daß nach der Messung des HDL-Cholesterins Detergens und/oder Cholesterinesterase in solcher Menge zugesetzt werden, daß das gesamte, in der Probe enthaltene Cholesterin unter $H_2O_2$-Bildung oxidiert wird und der Endwert der Farbreaktion als Maß für den Gesamtgehalt an Cholesterin gemessen wird.

**14.** Reagenz zur spezifischen Bestimmung des Cholesterins der HDL-Fraktion in Gegenwart der LDL-Fraktion der Lipoproteine des Serums enthaltend
Cholesterinesterase,
Cholesterinoxidase,
Tensid der Gallensäuregruppe,
nichtionisches Detergens,
Puffer-pH 5 bis 9 und
ein System zur photometrischen Bestimmung von $H_2O_2$,
**dadurch gekennzeichnet,**
daß es Cholesterinesterase aus Pankreas in einer Konzentration von 0,05 bis 30 U/ml, die Cholesterinoxidase in einer Konzentration von 0,1 bis 50 U/ml, Detergens der Gallensäuregruppe in einer Konzentration von 1,0 bis 20 mmol/l und nichtionisches Detergens in einer Konzentration von 0,1 bis 10 g/l, jeweils bezogen auf die im Test eingesetzte Verdünnung, enthält.

**15.** Reagenz nach Anspruch 14, **dadurch gekennzeichnet,** daß es Antikörper gegen LDL oder/und Apolipoprotein B enthält.

**16.** Reagenz nach Anspruch 14 oder 15, **dadurch gekennzeichnet,** daß es auf oder in einem blattförmigen Trägermaterial imprägniert vorliegt.

**17.** Reagenz nach Anspruch 16, **dadurch gekennzeichnet,** daß das Trägermaterial ein saugfähiges, quellfähiges oder filmbildendes Material ist.

## Claims

**1.** Process for the specific determination of the cholesterol of the HDL fraction in the presence of the LDL

fraction of the lipoproteins of the serum by action of cholesterol esterase for the liberation of the cholesterol and oxidation of the liberated cholesterol with cholesterol oxidase and oxygen with the formation of $H_2O_2$ and kinetic measurement of the $H_2O_2$ formation or of the oxygen consumption, characterised in that one uses the cholesterol esterase from pancreas and that the measurement is carried out within 2 minutes to 15 minutes after the start of the oxidase reaction at a temperature of 20 to 40° C during a predetermined time interval and during the measurement there is maintained in the reaction solution a cholesterol esterase concentration of 0.05 to 30 U/ml, a cholesterol oxidase concentration of 0.1 to 50 U/ml, a concentration of a tenside of the bile acid group of 1.0 to 20 mMol/l, a concentration of a non-ionic detergent of 0.1 to 10 g/1 and a pH value of 5 to 9.

**2.** Process according to claim 1, characterised in that the non-ionic detergent is added after the addition of the oxidase but 1 to 14 minutes before the measurement.

**3.** Process according to claim 1 or 2, characterised in that the measurement is carried out within 3 to 10 minutes after the start of the oxidase reaction.

**4.** Process according to claim 1 or 2, characterised in that a cholesterol esterase concentration of 0.05 to 10 U/ml is maintained.

**5.** Process according to claim 1 or 2, characterised in that a cholesterol oxidase concentration of 1 to 30 U/ml is maintained.

**6.** Process according to claim 1 or 2, characterised in that a concentration of tenside of the bile acid group of 1.5 to 8 mMol/l is maintained.

**7.** Process according to claim 1 or 2, characterised in that a concentration of non-ionic tenside of 0.4 to 4 g/l is maintained.

**8.** Process according to one of the preceding claims, characterised in that it is measured at a temperature of from 25 to 37° C.

**9.** Process according to one of the preceding claims, characterised in that sodium cholate is used as tenside of the bile acid group.

**10.** Process according to one of the preceding claims, characterised in that a polyethylene oxide group-containing tenside is used as non-ionic tenside.

**11.** Process according to one of the preceding claims, characterised in that the reaction is carried out in the presence of anti-LDL-antibodies and/or of antiapolipoprotein B-antibodies.

**12.** Process according to one of the preceding claims, characterised in that, in addition, within the first two minutes after the start of the oxidase reaction, the $H_2O_2$ formation is determined kinetically as measure of the cholesterol bound in the LDL fraction of the serum.

**13.** Process according to one of the preceding claims, characterised in that, after the measurement of the HDL cholesterol, detergent and/or cholesterol esterase are added in such amount that the total cholesterol contained in the sample is oxidised with the $H_2O_2$ formation and the end value of the colour reaction is measured as measure of the total content of cholesterol.

**14.** Reagent for the specific determination of the cholesterol of the HDL fraction in the presence of the LDL fraction of the lipoproteins of the serum containing cholesterol esterase, cholesterol oxidase, tenside of the bile acid group, non-ionic detergent, buffer pH 5 to 9 and a system for the photometric determination of $H_2O_2$, characterised in that it contains cholesterol esterase from pancreas in a concentration of 0.05 to 30 U/ml, the cholesterol oxidase in a concentration of 0.1 to 50 U/ml, detergent of the bile acid group in a concentration of 1.0 to 20 mMol/l and the non-ionic detergent in a concentration of 0.1 to 10 g/l, in each case referred to the dilution used in the test.

**15.** Reagent according to claim 14, characterised in that it contains antibodies against LDL and/or

apolipoprotein B.

**16.** Reagent according to claim 14 or 15, characterised in that it is present impregnated on or in a leaf-like carrier material.

**17.** Reagent according to claim 16, characterised in that the carrier material is an absorbent, swellable or film-forming material.

**Revendications**

**1.** Procédé de dosage spécifique du cholestérol de la fraction HDL en présence de la fraction LDL des lipoprotéines du sérum, par action de cholestérol estérase qui libère le cholestérol et oxydation du cholestérol libéré avec de la cholestérol oxydase et de l'oxygène avec formation de $H_2O_2$,et mesure cinétique de la formation de $H_2O_2$ ou de la consommation d'oxygène, caractérisé en ce qu'on utilise la cholestérol estérase du pancréas, en ce qu'on effectue la mesure dans les 2 minutes à 15 minutes qui suivent le démarrage de la réaction avec l'oxydase, à une température de 20 à 40°C, pendant un intervalle de temps prédéterminé, et en ce que pendant la mesure on maintient dans la solution réactionnelle une concentration de cholestérol estérase de 0,05 à 30 U/ml, une concentration en cholestérol oxydase de 0,1 à 50 U/ml, une concentration en un agent tensio-actif du groupe des acides biliaires de 1,0 à 20 mmoles/litre, une concentration en détergent non ionique de 0,1 à 10 g/l et une valeur de pH de 5 à 9.

**2.** Procédé selon la revendication 1, caractérisé en ce qu'on ajoute le détergent non ionique après l'addition de l'oxydase, mais 1 à 14 minutes avant la mesure.

**3.** Procédé selon la revendication 1 ou 2, caractérisé en ce que la mesure est effectuée dans les 3 à 10 minutes qui suivent le démarrage de la réaction avec l'oxydase.

**4.** Procédé selon la revendication 1 ou 2, caractérisé en ce qu'on maintient une concentration en cholestérol estérase de 0,05 à 10 U/ml.

**5.** Procédé selon la revendication 1 ou 2, caractérisé en ce qu'on maintient une concentration en cholestérol oxydase de 1 à 30 U/ml.

**6.** Procédé selon la revendication 1 ou 2, caractérisé en ce qu'on maintient une concentration en agent tensio-actif du groupe des acides biliaires de 1,5 à 8 mmoles/l.

**7.** Procédé selon la revendication 1 ou 2, caractérisé en ce qu'on maintient une concentration en agent tensio-actif non ionique comprise de 0,4 à 4 g/l.

**8.** Procédé selon l'une des revendications précédentes, caractérisé en ce qu'on effectue la mesure à une température de 25 à 37°C.

**9.** Procédé selon l'une des revendications précédentes, caractérisé en ce qu'on utilise du cholate de sodium comme agent tensio-actif du groupe des acides biliaires.

**10.** Procédé selon l'une des revendications précédentes, caractérisé en ce qu'on utilise un agent tensio-actif contenant des groupes polyoxyéthylène comme agent tensio-actif non ionique.

**11.** Procédé selon l'une des revendications précédentes, caractérisé en ce que la réaction est mise en oeuvre en présence d'anticorps anti-LDL ou/et d'anticorps antiapolipoprotéine B.

**12.** Procédé selon l'une des revendications précédentes, caractérisé en ce que, en plus, dans les deux premières minutes suivant le démarrage de la réaction avec l'oxydase, on détermine de façon cinétique la formation de $H_2O_2$ comme mesure du cholestérol lié dans la fraction LDL du sérum.

**13.** Procédé selon l'une des revendications précédentes, caractérisé en ce que, après la mesure du HDL cholestérol, on ajoute du détergent et/ou de la cholestérol estérase en une quantité telle que le

cholestérol total contenu dans l'échantillon soit oxydé avec formation de $H_2O_2$ et en ce qu'on mesure la valeur finale de la réaction de coloration comme mesure de la teneur totale en cholestérol.

**14.** Réactif pour le dosage spécifique du cholestérol de la fraction HDL en présence de la fraction LDL des lipoprotéines du sérum, contenant:
une cholestérol estérase,
une cholestérol oxydase,
un agent tensio-actif du groupe des acides biliaires,
un détergent non ionique,
un tampon de pH 5 à 9 et
un système pour le dosage photométrique de $H_2O_2$, caractérisé en ce qu'il contient une cholestérol estérase de pancréas à une concentration de 0,05 à 30 U/ml, la cholestérol oxydase à une concentration de 0,1 à 50 U/ml, un détergent du groupe des acides biliaires à une concentration de 1,0 à 20 mmoles/l et un détergent non ionique à une concentration de 0,1 à 10 g/l, rapportées à chaque fois à la dilution utilisée dans l'essai.

**15.** Réactif selon la revendication 14, caractérisé en ce qu'il contient des anticorps contre les LDL ou/et contre l'apolipoprotéine B.

**16.** Réactif selon la revendication 14 ou 15, caractérisé en ce qu'il imprègne la surface ou l'intérieur d'une matière support en forme de feuilles.

**17.** Réactif selon la revendication 16, caractérisé en ce que la matière support est une matière absorbante, susceptible de gonfler ou filmogène.

FIG. 1

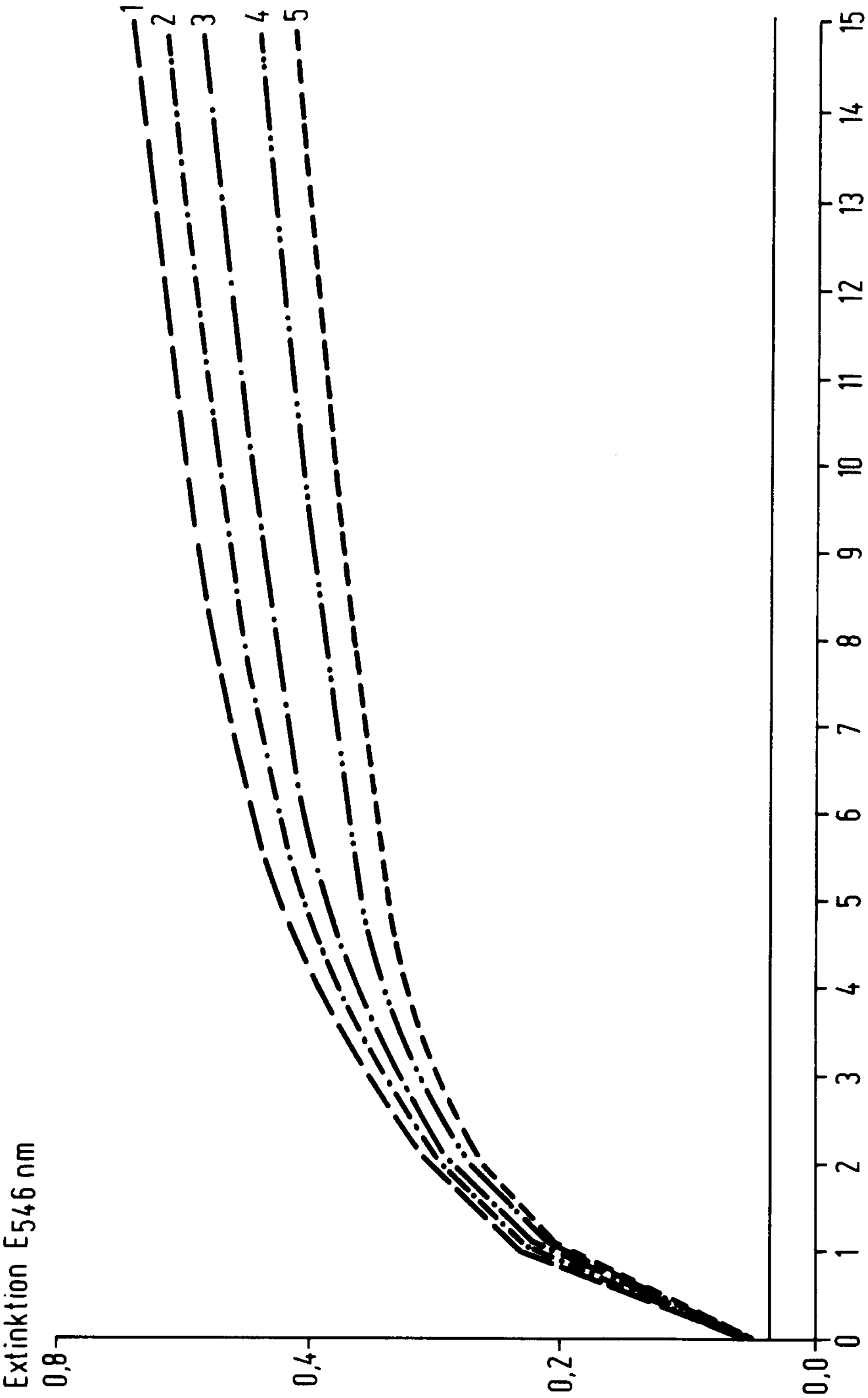
Extinktion $E_{546\,nm}$
0,8
0,4
0,2
0,0
0
1
2
3
4
5
6
7
8
9
10
11
12
13
14
15
Zeit [Minuten]
1
2
3
4
5

FIG. 2

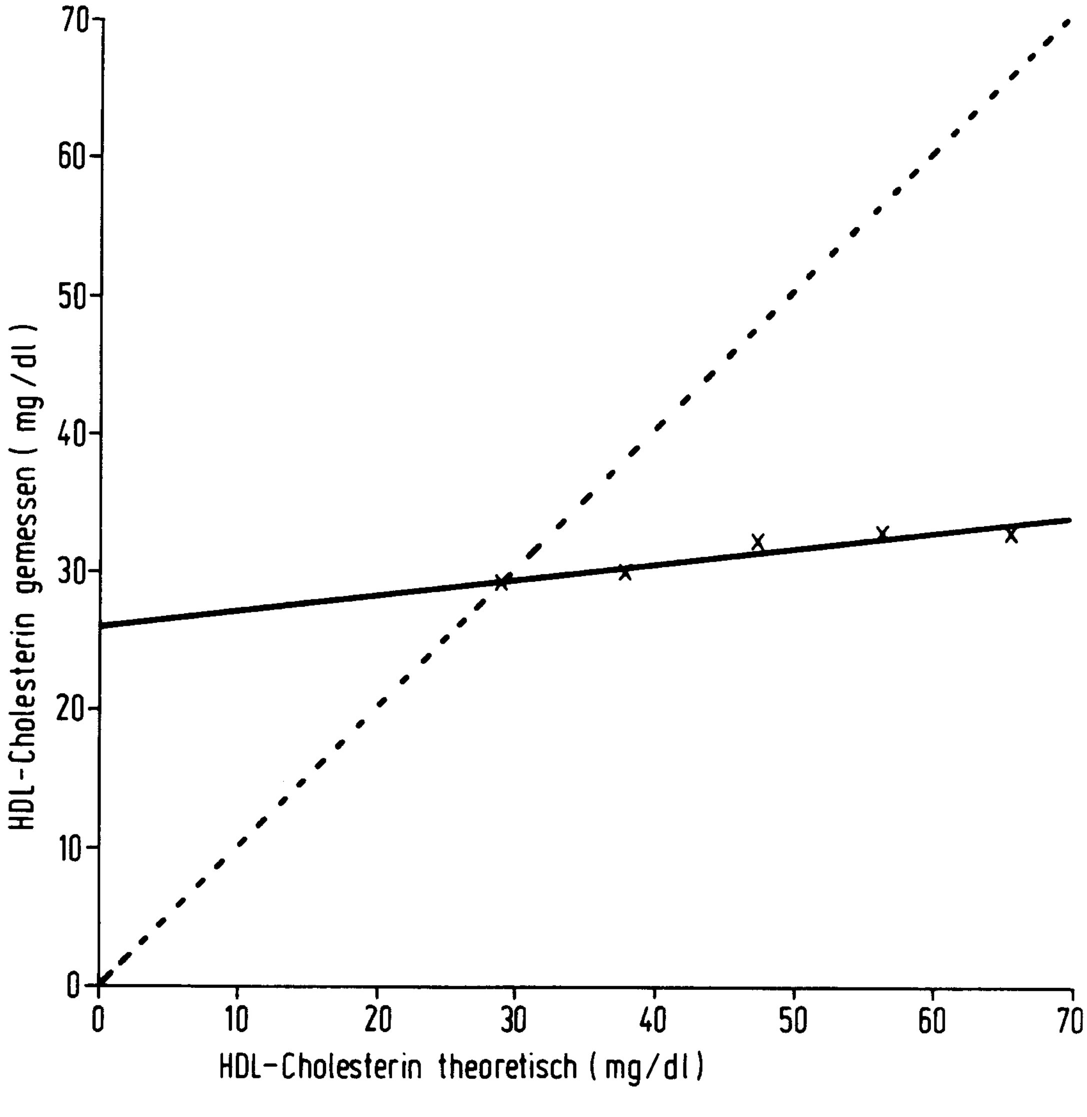
70
60
50
40
30
20
10
0
0
10
20
30
40
50
60
70
HDL-Cholesterin gemessen ( mg/dl )
HDL-Cholesterin theoretisch ( mg/dl )

FIG. 3

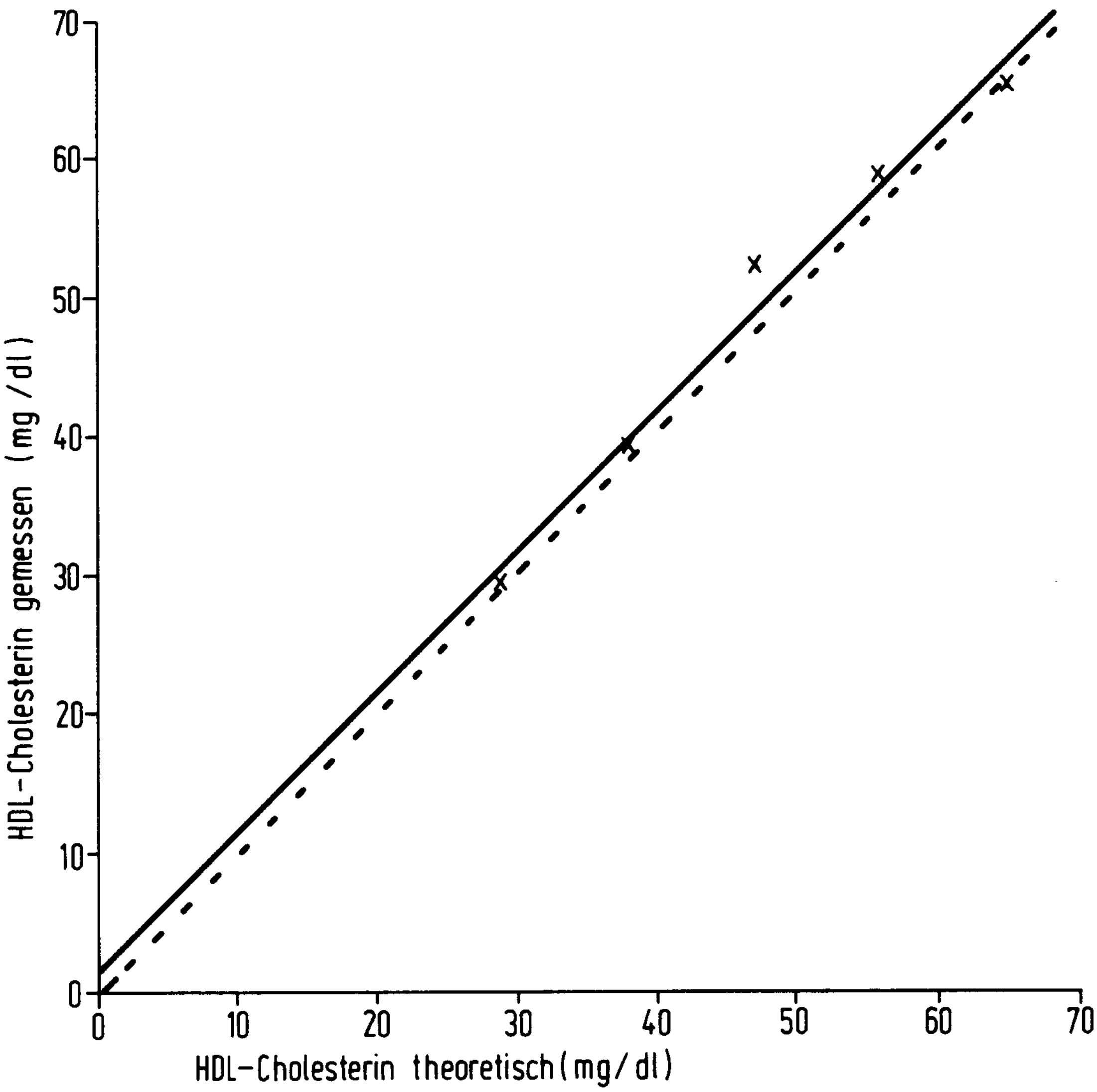
70
60
50
40
30
20
10
0
HDL-Cholesterin gemessen (mg/dl)
0
10
20
30
40
50
60
70
HDL-Cholesterin theoretisch (mg/dl)

FIG. 4

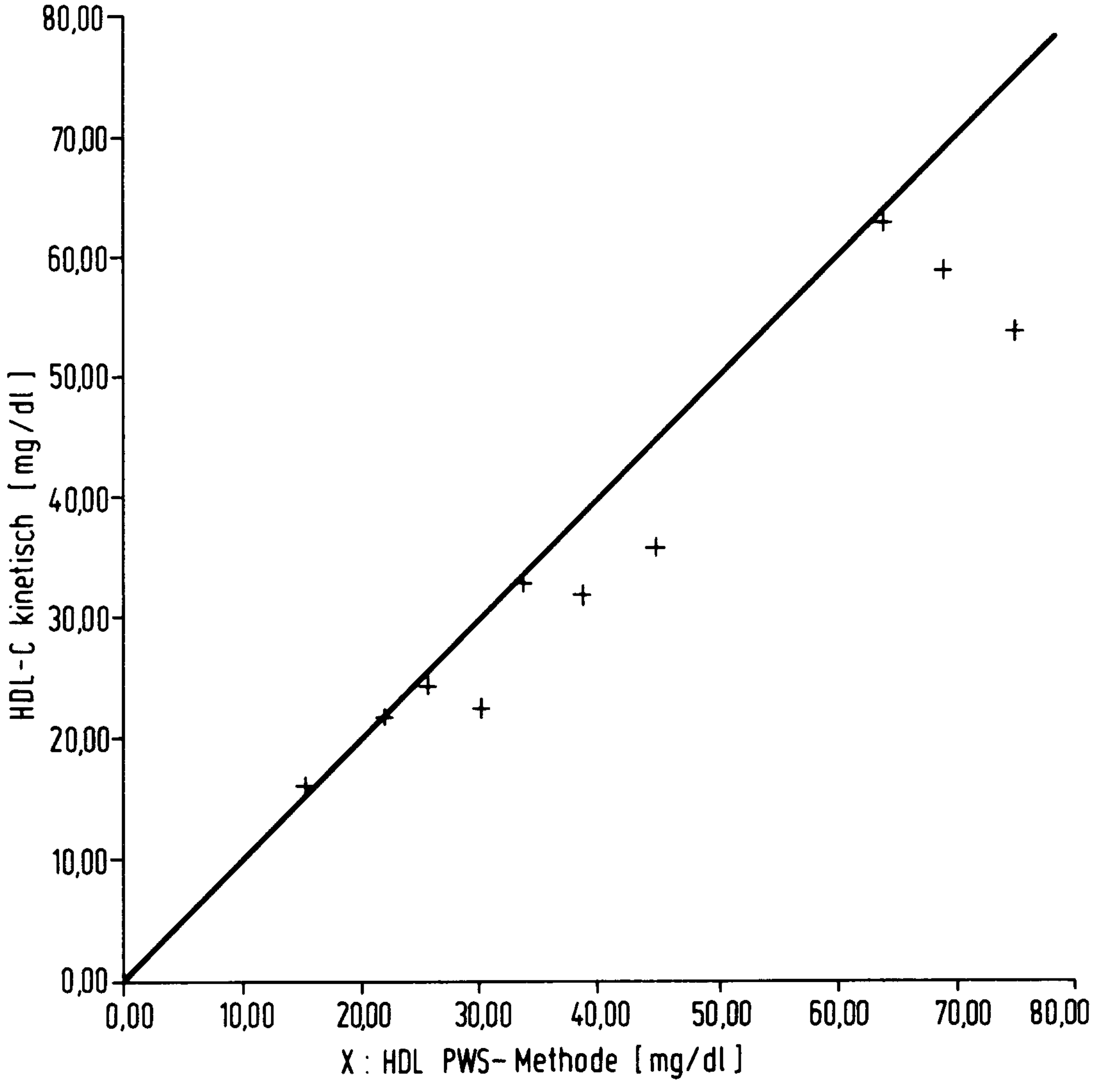
80,00
70,00
60,00
50,00
40,00
30,00
20,00
10,00
0,00
HDL-C kinetisch [mg/dl]
0,00
10,00
20,00
30,00
40,00
50,00
60,00
70,00
80,00
X : HDL PWS-Methode [mg/dl]

FIG. 5

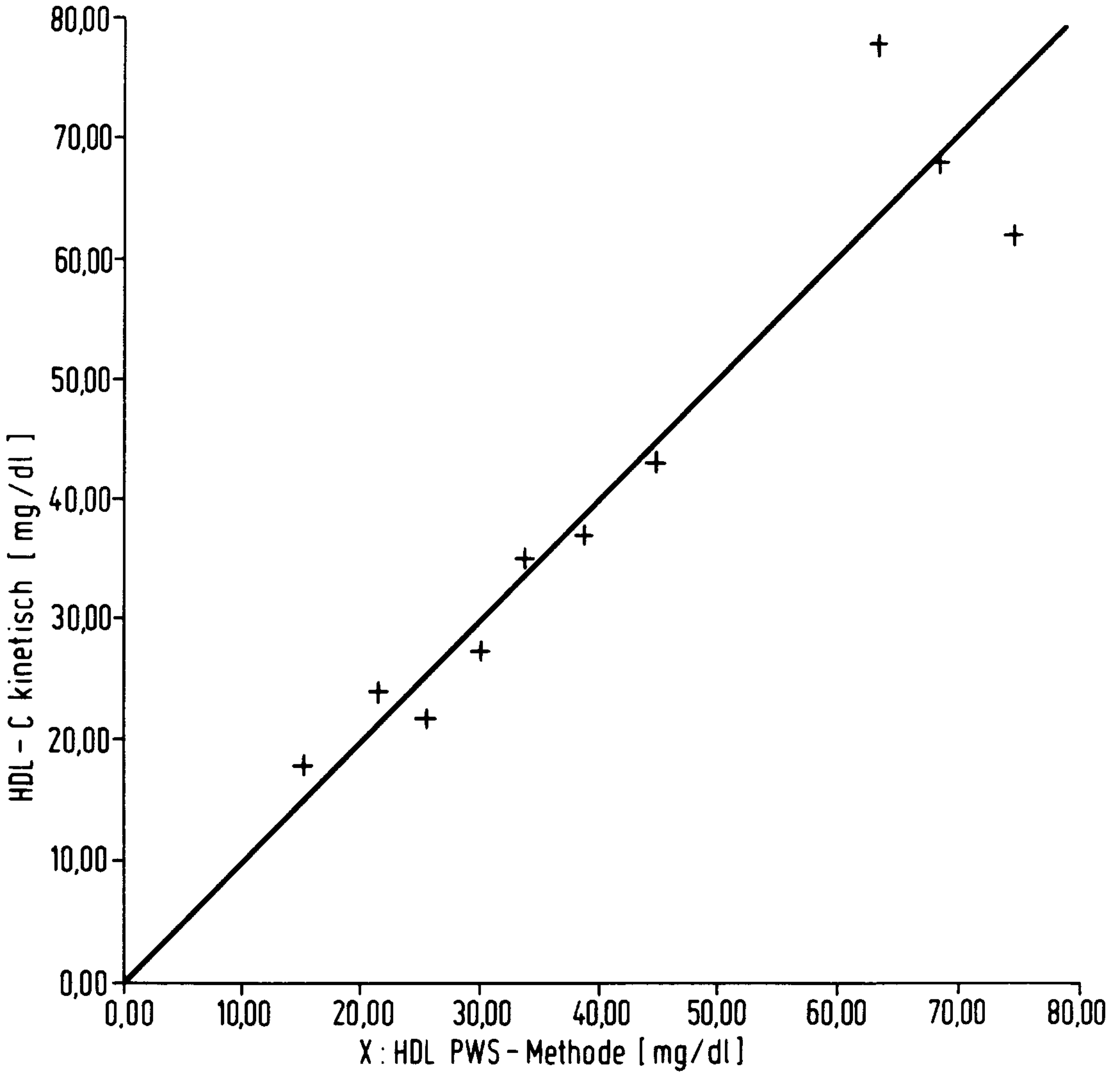
80,00
70,00
60,00
50,00
40,00
30,00
20,00
10,00
0,00
HDL - C kinetisch [ mg/dl ]
0,00
10,00
20,00
30,00
40,00
50,00
60,00
70,00
80,00
X : HDL PWS - Methode [ mg/dl ]